# EUROPEAN PATENT APPLICATION

(11) **EP 2 476 755 A1**
(43) Date of publication of application: **18.07.2012**
(21) Application number: 11151186.1
(22) Date of filing: 17.01.2011
(51) Int. Cl.: C12N 15/85, C12N 5/10

(54) **S/MAR containing expression vectors, cell and cell lines as well as methods using the same**

(71) Applicant: Helmholtz-Zentrum für Infektionsforschung GmbH, 38124 Braunschweig (DE)
(72) Inventor: Wirth, Manfred, 38300, Wolfenbüttel (DE)
(74) Representative: Gerstein, Hans Joachim

(57) **Abstract**

The present invention relates generally to newly discovered S/MAR elements useful in expression vectors. In particular, the present invention relates to the use of the MUR-region of CMV as S/MAR element in expression vectors. In another aspect, the present invention relates to vector nucleic acids containing the same as well as host or host cells containing said vector nucleic acids either stably integrated into the host genome or episomally replicating, or alternatively, present as a minicircle expression vector for stable or a transient expression. In another aspect, the present invention relates to a method for the production of a molecule of interest based on the MUR-region of CMV as a S/MAR region as well as kits or systems containing the vector nucleic acid or cell or cell line according to the present invention.

SEQUENCE LISTING

<110>  Helmholtz-Zentrum für Infektionsforschung GmbH

<120>  S/MAR containing expression vectors, cell and cell lines as well
       as methods using the same

<130>  3275-029-EP-1

<160>  5

<170>  PatentIn version 3.3

<210>  1
<211>  591
<212>  DNA
<213>  human cmv

<400>  1
ctgcagtgaa taataaaatg tgtgtttgtc cgaaatacgc gtttgagatt tctgtcccga      60

ctaaattcat gtcgcgcgat agtggtgttt atcgccgata gagatggcga tattggaaaa     120

atcgatattt gaaaatatgg catattgaaa atgtcgccga tgtgagtttc tgtgtaactg     180

atatcgccat ttttccaaaa gttgattttt gggcatacgc gatatctggc gatacgctta     240

tatcgtttac gggggatggc gatagacgcc tttggtgact tgggcgattc tgtgtgtcgc     300

aaatatcgca gtttcgatat aggtgacaga cgatatgagg ctatatcgcc gatagaggcg     360

acatcaagct ggcacatggc caatgcatat cgatctatac attgaatcaa tattggccat     420

tagccatatt attcattggt tatatagcat aaatcaatat tggctattgg ccattgcata     480

cgttgtatcc atatcataat atgtacattt atattggctc atgtccaaca ttaccgccat     540

gttgacattg attattgact agttattaat agtaatcaat tacggggtca t              591


<210>  2
<211>  686
<212>  DNA
<213>  simian cmv

<400>  2
tggcacggtg ccaagaaaat ccggatttgg caccgagcca aaaaaacttg gcaacgtgcc      60

aaatccaaaa tggcgacttg gcactgtgcc aacaaagatg gcggatctgg cacggtgcca     120

attgcaaatg gcgacttggc attgagccaa ttcaatatgg cggacttgga cttgggccaa     180

ttcaaaatgg tggacctgga tttctgccaa atcaaaatgg cgattttgga tactggccaa     240

ttcaatatgg cgaccatgga tctgtgccaa ttcaatatgg aggacctgga ccatagccaa     300

ttcaatatgg cgtatatgga ctcatgccaa ttcaatatgg tggatctgga cctgtgccaa     360

ttcaatatgg cgtatatgga ctcgtgccaa ttcaatatgg tggatctgga ccccagccaa     420

ttcaatatgg cggacttggc accatgccaa ttcaatatgg cggacctggc actgtgccaa     480

ctggggaggg gtctacttgg cacggtgcca agtttgagga ggggtcttgg ccctgtgcca     540

agtccgccat attgaattgg catggtgcca ataatggcgg ccatattggc tatatgccag     600

gatcaatata taggcaatat ccaatatggc cctatgccaa tatggctatt ggccaggttc     660

aatactatgt attggcccta tgccat                                          686

<210>  3
<211>  600
<212>  DNA
<213>  mice cmv

<400>  3
gttaacgtag gtagggtcgt atcgagccgc ggtccgtcct gctctgggct cgaatggcat      60

gggggacagc aattatatgg ttaactccgc ccgttttatg actagaacca atagttttta     120

atgccaaatg cactgaaatc ccctaatttg caaagccaaa cgccccctat gtgagtaata     180

cggggacttt ttacccaatt tcccacgcgg aaagccccct aatacactca tatggcatat     240

gaatcagcac ggtcatgcac tctaatggcg gcccataggg actttccaca tagggggcgt     300

tcaccatttc ccagcatagg ggtggtgact caatggcctt tacccaagta cattgggtca     360

atgggaggta agccaatggg tttttcccat tactggcaag cacactgagt caaatgggac     420

tttccactgg gttttgccca agtacattgg gtcaatggga ggtgagccaa tgggaaaaac     480

ccattgctgc caagtacact gactcaatag ggactttcca atgggttttt ccattgttgg     540

caagcatata aggtcaatgt gggtgagtca atagggactt tccattgtat tctgcccagt     600

<210>  4
<211>  729
<212>  DNA
<213>  rat cmv

<400>  4
atatttttat ggaaatttta aaaaattctg gtaagctatt taaaaaaatg aactttatta      60

tgaaactatt gcccttttct ctaaaaaaca acacaatttc acggaatatc ctatgattaa     120

ttatgacctt ttagccagtt cccatattaa gaatgagtta tagatgactc tctttaaaaa     180

attattcgat ttaaaccatc tgttttaaag cacagcattt gtgaataatg tgaagaactt     240

agaagtataa tctactccaa ggtctgatgt atttttcaag gccacgttaa agtgtatgct     300

tgtaacagag tgcttacatt caagccaaat gttaatataa caatcctgaa ttcgtacata     360

atgtgaataa gacactcaac tctatttaaa tccagatcta aatagttact tttatctaaa     420

tgtcaccatc tgtttctact tagaataata aacttcttaa aggtcacgta tcgggctgat     480

tataaatcat tataattata acaaaacaga tgatttgttt aaaggtcaca tcccgttccg     540

tggtcttttt agtcgaaata actattaatc ttcattatgt ttctgagaaa gtttaaatat     600

cacgatttcc acccataaca gtcattatga gtcagtggga gtcatactga atcagggtat     660

tttaactgga atttttttga aaaacatgag tttttcttaa ggtcaacatc tggtcttata     720

aacagaact                                                             729

<210>  5
<211>  720
<212>  DNA
<213>  guinea pig cmv

<400> 5
ccgggacttt ccatatcatc caaattaatc aataatgatt acaagcggac aggttgttgg 60

catctagtta ttcaggaaaa tccatatgcg tgctgccaac atcaaaacaa tgtaatatat 120

tcatgagatc atgattaatt taatggaaaa cacctaaaaa atccagtcat catctggaaa 180

gcacctaacg ttacgtaaaa ttttaatatg attcagagat gggccgggtt atacggaata 240

cgcctataaa agaggaggag ttcgctggtt tagaatcagt attgtgccag actccgaaga 300

ggacacatct cccgtgctcg gaatgctgcc aatatattaa aagaataggt gcgtatggtt 360

atctttgata tagcacaggt agaatacgtg tatggaggtg acctttacct gtgagagtag 420

gttagtaaac aaagaatcgt gccagactga aggtacagca agtcaattta tatgtgatag 480

ttaataatat agattacatt gaaccgatat tgtgtagttt gacgtggaat gtaggtttgc 540

ttactagatg atcgatagcg caggcttata gcgtaagaga tgtgatagat gcgatggtat 600

gcatcggcag tttccgacag atgttcgtac tgaattgttc taataataaa aatgacttca 660

cgtccatata agtctcccta gtttttctta ttggatgact gtcgagaagg cggtaacatg 720

## Description

The present invention relates generally to newly discovered S/MAR elements useful in expression vectors. In particular, the present invention relates to the use of the MUR-region of CMV as S/MAR element in expression vectors. In another aspect, the present invention relates to vector nucleic acids containing the same as well as host or host cells containing said vector nucleic acids either stably integrated into the host genome or episomally replicating, or alternatively, present as a minicircle expression vector for stable or a transient expression. In another aspect, the present invention relates to a method for the production of a molecule of interest based on the MUR-region of CMV as a S/MAR region as well as kits or systems containing the vector nucleic acid or cell or cell line according to the present invention.

### Background Art

Gene transfer technology in mammalian cells are diverse and include various approaches including *in vitro* expression in cells engineered for production of proteins of pharmaceutical/industrial interest or with altered metabolism and in vivo expression by gene transfer. While viral vectors are the most sufficient vehicles for gene transfer, the use is restricted by major limiting factors, such as the occurrence of immune responses to viral components and interference with cellular integrity. In a case of retroviral systems, the danger of insertion mutagenesis into host genome has become apparent, shifting the focus to non-integrating episomal systems. Prototypes are found in viral systems which rely on origins of replication (oris), e.g. described from the SV40, BK virus, BPV-1 or EBV. The function of oris is supported by elements mediating retention in the nucleus of the host cell.

The level and pattern of expression often differ among different transforming cells and/or tend to decline with time. This variability of expression and rapid extinction are manifestations of the structure effects such as gene elimination or epigenetic silencing and the influence of dominant regulatory elements flanking the side of integration of the transgene.

Further, transient gene expression is an increasingly applied method for rapid production of small amounts of pharmaceutical proteins, especially for evaluation purposes and toxicity testing, without the need to establish a new cell line. During transient transfection, the genetic material needed for expression of a target protein is incorporated into the cells's nucleus while avoiding its integration into the genome. Typically, the transfer is affected by the help of viral vectors or alternatively, the use of non-viral vectors, such as polyethylenimine.

Recently, non-viral replicating and non-integrating minicircles that are based on the benefitial self-organizational and regulatory properties of scaffold or matrix-attached regions (S/MAR) have been described. Broll S., et al., 2010, J. Mol. Biol. 395, 950-965 describe minicircle performance depending on S/MAR-nuclear matrix interactions. S/MAR are one type of DNA elements described to minimize silencing by regulatory elements at the side of integration into the genome or in episomes. Moreover, S/MARs can provide position-independent expression of integrated transgenes. MARs are defined as the genomic DNA sequences at which the chromatin is anchored to the nuclear matrix proteins during interphase.

The minicircles described in the art are constructs of approximately 4 kb sizes established by excision of prokaryotic parts with only one remaining active transcription unit. Minicircles utilize the replication machinery of the host cell due to the association with the nuclear matrix and are not affected by epigenetic silencing.

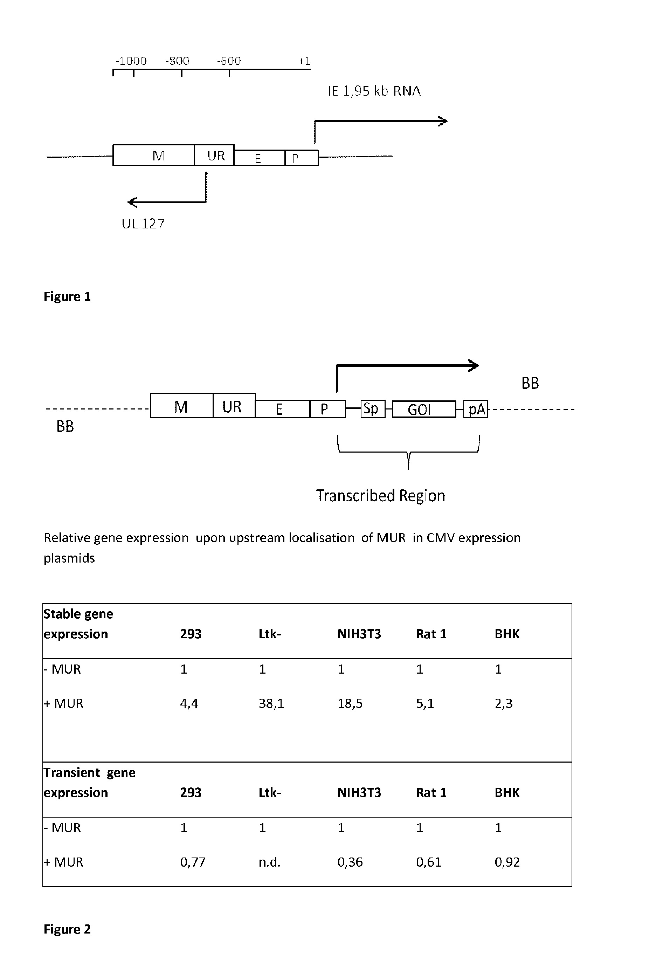

The human cytomegalomovirus (CMV) immediate early promoter/enhancer is known since 1985 for use in the expression of genes in mammalian cells or cell lines. This promoter/enhancer shows high transcription efficiency and is applied in different approaches for protein production in mammalian cells. The CMV immediate early promoter/enhancer is a 600 bp element consisting of the promoter and enhancer of CMV. Upstream of the CMV immediate early promoter/enhancer region an additional region, -1145 to -610, has been described having a modulator region, M, -1145 to -780, and a so called unique region (UR), -780 to -610 whereby + 1 is the transcription starting point, e.g. see figure 1. It is described that this MUR region is effective as a bidirectional regulator of gene expression of the immediate early genes of CMV and the open reading frame UL127 in opposite direction. The unique region contains binding sides for various transcription factors, like NF1, CTP, SATPB1, Fox A, or suppressor of hairy wing. This region mediates repression of transcription of the upstream UL127 open reading frame. The modulator is responsible for different expression in differentiated and non-differentiated cells. It has been demonstrated so far that the M or UR region in cell systems for protein expression based on transient or stable gene expression influence of M or UR is not given or very low.

As mentioned above, S/MAR elements mediate site independent expression in stably transfected cells since these elements protect transcription units integrated into the genome of the cells from chromosomal environment. In addition, it is described that S/MARs represents borderlines for active and inactive chromatin. Typically, S/MARs do not influence transient expression. However, recently, Broll et. al. describes minicircle performance depending on S/MAR nuclear matrix interactions in CHO expression systems showing that maintenance and stable expression depends on the cell cycle phase during gene transfer. In minicircles S/MAR mediate attachment to the nuclear matrix in stable clones and, thus, mediate maintenance in the nucleus. Further, episomal replication and enhanced stable expression also in the absence of a selection marker are known.

An object of the present invention is to provide new vector nucleic acid molecules allowing transient as well as stable expression of molecules of interest in host cells. In addition, a need for new elements allowing to increase gene expression in stable transfected cells as well as allowing to influence transient gene expression, in particular, when using episomal minicircles.

### Brief summary of the present invention

In a first aspect, the present invention relates to the use of the MUR-region of CMV as S/MAR elements in expression vectors. That is, the present inventors recognized that the MUR-region (modulator and unique region) of CMV (cytomegalovirus) acts as S/MAR element in expression vectors suitable both for transient as well as stable expression. In particular, the present inventors recognize that the MUR of rodent, simian or human CMV, in particular, human CMV region represents a new S/MAR element. Thus, it is possible to increase and extend productivity of transient expression when placed into a specific vector location as well as productivity in stable transfection.

Preferably, the MUR-region is the nucleic acid sequence of Seq.ID No. 1.

In addition, the present invention relates to a vector nucleic acid suitable for expressing at least one molecule of interest in a mammalian cell, comprising the S/MAR-region consisting of the MUR-region of CMV, a promoter and the molecule of interest or a gene encoding the molecule of interest.

Using this vector nucleic acid allows to increase expression of the molecule of interest in stably transfected cell lines, in particular, in parental plasmids, when MUR located 5' or is flanking the transcription unit as well as extending the transient gene expression in a cell line, in particular in MCs if MUR is part of the transcription unit.

In a further aspect, the present invention relates to cells or cell lines containing the vector nucleic acid according to the present invention. Said cell lines are able to express the molecule of interest transiently or stably and may contain said vector of interest integrated in the host genome or as an episomally replicating vector or as a minicircle expression vector.

Further, the present invention relates to a method for the production of a molecule of interest, in particular, a gene of interest or a polypeptide of interest comprising the step of transfecting a cell or cell line, preferably, a mammalian cell or cell line, with an expression vector containing the MUR-region of CMV as S/MAR region. Preferably, said vector is a vector nucleic acid according to the present invention.

In another aspect, the present invention relates to a kit or system containing the vector nucleic acid according to the present invention or a cell or cell line according to the present invention. Said kit or system is particularly useful in the production of recombinant molecules of interest, in particular, nucleic acid molecules of interest or polypeptides of interest.

### Brief description of the drawings

Figure 1: Abb. 1 Schematic drawing of the hCMV major immediate early promoter and the dual regulatory region (M = modulator, UR= unique region) upstream in the genome of the human cytomegalovirus. The M + UR guides the transcription of the UL127 open reading frame upstream and influences the gene expression of the immediate early genes downstream
Figure 2: Relative gene expression in transiently and stably transfected animal cell lines of expression vectors carrying or devoid of an upstream MUR.

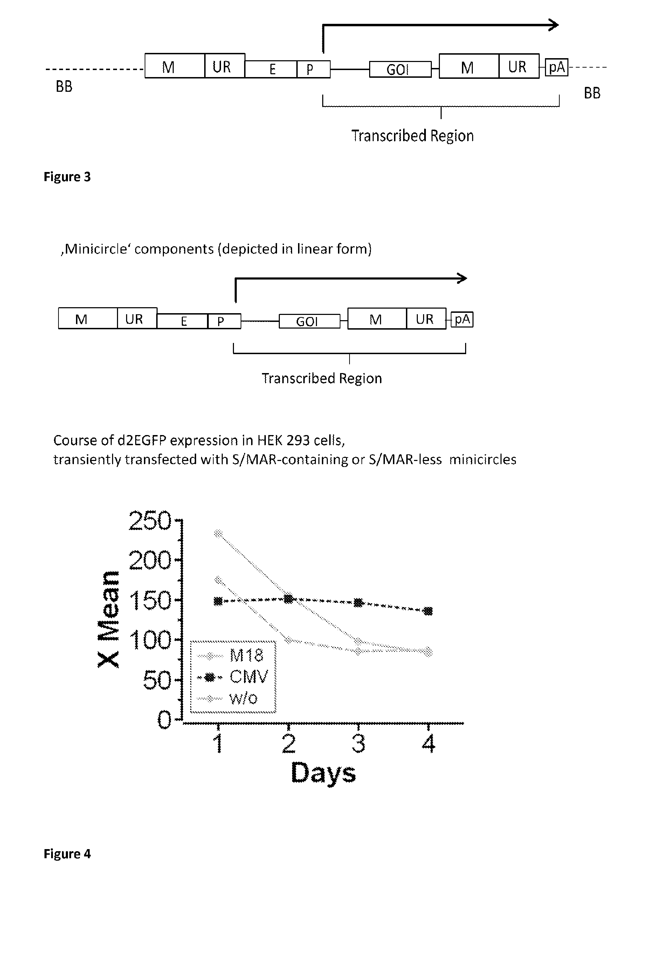

Figure 3: Parental plasmids as precursors for the generation of MUR-minicircles. The parental plasmid carries a bacterial transcription unit (BB = bacterial backbone, dashed). For ease the linear form the parental plasmid is shown. The eukaryotic transcription unit consisting of promoter with 5' regulatory upstream region (e.g. the hCMVIE or SV40 promoter preceded by a MUR ) a gene of interest (GOI), a poly A signal and a MUR located between GOI and pA within the transcribed region. BB and eukaryotic unit are separated by two FRT sites (not shown), representing the recognition target for yeast FLP recombinase.
Figure 4: Schematic drawing of a minicircle derived from recombination of the parental plasmid after expression of FLP recombinase and purification. The MC consists of the eukaryotic transcription unit including the transcribed MUR. Transcription is thought to allow opening of the MUR double strand to exert the S/MAR activities.

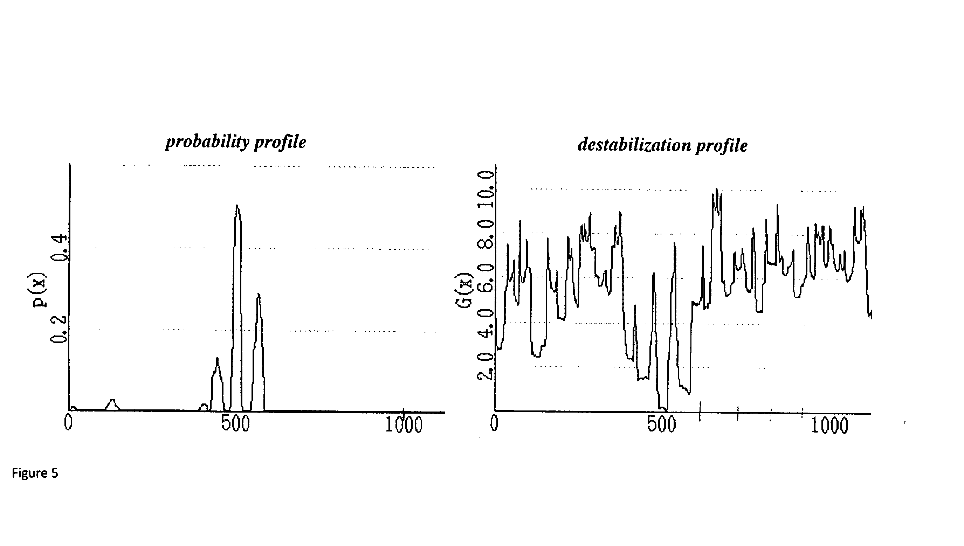

Figure 5: Analysis of stress induced duplex destabilization (SIDD) (Benham et al., 1997, J. Mol Biol., 274; 181-196) identifies S/MARs in the upstream region of various animal cytomegaloviruses.

### Detailed description of the present invention

The present inventors recognized that the MUR-region (modulator and unique region) of CMV (cytomegaloma virus) has S/MAR element properties. That is, the present inventors recognized that the MUR-region of CMV is able to influence stable as well as transient expression of molecules of interest in cell or cell lines wherein said MUR region is used as S/MAR elements in vector nucleic acid molecules.

Hence, in a first aspect, the present invention relates to the use of the MUR-region of CMV as S/MAR element in expression vectors.

Particularly preferred, said use is directed to expression vectors for transient expression or, alternatively, for stable integration into the host chromosome and/or stable expression upon extrachromosonal replication.

In a preferred embodiment, the S/MAR is a MUR-region of rodent, simian or human CMV. Particularly preferred, the S/MAR region is the MUR-region of human CMV, like the MUR-region according to Seq.ID NO. 1. GenBank X03922 entry, -1145 to -610 shows the MUR-region of human CMV, of course, other hCMV related isolates exists containing variations, like substitutions, deletions or additions in the sequence. It is contemplated that said variations in the MUR-region are within the meaning of the term "MUR-region of CMV. Further, it is preferred that the MUR-region useful as S/MAR is derived from simian CMV, e.g. as described in genbank Acc. No. U38308.1, position 3584-4270; MUR of mouse CMV,Genbank Acc. No. L06570, position 1-601; rat CMV e.g as described in Genbank Acc. No. U62396, postion 1-721; or guinea pig e.g. as described in Genbank Acc. No. FJ355434, position 195520-194801.

According to the present invention, the term MUR-region refers to the modular and unique region of CMV. An example of said region is the nucleic acid sequence of Seq.ID No. 1 representing the MUR-region of human CMV. Another preferred embodiment is the MUR region of simian CMV (Seq. ID. No. 2), mouse CMV (Seq. ID No. 3), rat CMV (Seq. ID. No. 4) and guinea pig CMV (Seq. ID No. 5).

The term "S/MAR element" as used herein stands for scaffold/matrix attachment region and refers to DNA sequences conferring anchorage to the nuclear matrix of animal or plant cells.

The term "molecule of interest" refers to molecules, either on nucleic acid level or amino acid level.

By using the MUR-region of CMV as S/MAR element in expression vectors, it is possible to increase the expression level in stably transfected cells and cell lines at least two fold compared to expression vectors not containing the same. In addition, it is possible to extend the time of expression in transiently infected cells when using the S/MAR element compared to transient expression vectors not containing the same.

Thus, in a further embodiment of the present invention, a vector nucleic acid suitable for expressing at least molecule of interest in a mammalian cell, comprising a S/MAR region consisting of the MUR-region of CMV, in particular, of rodent, simian or human CMV, particularly preferred of human CMV, a promoter and the molecule of interest or a gene encoding the molecule of interest, is provided.

Said vector nucleic acid is suitable for transient as well as stable expression of the molecule of interest.

Preferably, the vector nucleic acid according to the present invention is a nucleic acid having the following order from the 5' end to the 3' end:
5'-S/MAR, promoter, gene encoding the molecule of interest or the molecule of interest, poly A, whereby the S/MAR may be present either at the 5' end or 3' end or both ends. The promoter is operably linked with the gene encoding the molecule of interest or the molecule of interest and the poly A tail is operably linked with the gene encoding the molecule of interest or the molecule of interest as well. The vector nucleic acid according to the embodiment described above is particularly suitable for stable expression of the molecule of interest.

In a preferred embodiment, the vector nucleic acid is a vector nucleic acid wherein the S/MAR region consisting of the MUR-region of CMV is present on both, the 5' end and the 3' end.

In another preferred embodiment of the vector nucleic acid according to the present invention, the vector nucleic acid has the following order from the 5' end to the 3' end:
5'-Promoter operably linked with the molecule of interest or the gene encoding the molecule of interest, S/MAR consisting of the MUR-region of CMV according to the present invention and the poly A tail, wherein the S/MAR and the poly A tail are operably linked with each other.

Upstream of the promoter region, a further MUR region may be present, preferably, derived from CMV.

The promoter/enhancer of the vector nucleic acid may be derived from any known and suitable promoter/enhancer described in the art. Preferably, the promoter/enhancer region is derived from SV40, CMV, ubiquitin C, or EF1alpha.

Further, the vector nucleic acid may contain prokaryotic parts. That is, the vector nucleic acids according to the present invention encompass constructs further containing prokaryotic elements allowing for amplification in a host, e.g. a prokaryotic host. These prokaryotic elements may be excised by suitable means in the transfected host to create so called minicircles composed of the vector nucleic acid containing the molecule of interest together with the MUR-region having a S/MAR functionality. Suitable systems are described e.g. in Nehlsen K., et al., 2006, Gene Ther. Mol. Biol, 10, 233-244.

The vector nucleic acid according to the embodiment of a vector nucleic acid having the order from the 5' end to the 3' end of 5'-promoter, a molecule of interest or a gene encoding the molecule of interest, S/MAR consisting of the MUR-region of CMV and poly A is particularly useful for transient expression as well as for stable expression. The transcribed region comprises the promoter region, the gene of interest region, the S/MAR and the poly A tail. This vector nucleic acid is particularly useful for forming minicircle expression vectors for transient expression. However, this vector nucleic acid allows also stable integration into the host genome or episomal replication.

The vector nucleic acid according to the present invention is particularly a vector nucleic acid wherein the molecule of interest is a gene encoding a poly peptide of interest, a gene encoding an antisense or silencer nucleic acid molecule, in particular, an RNA molecule, an antibody, a DNA vaccine, a regulatory RNA molecule, or a non-coding RNA molecule.

Moreover, the present invention provides the use of the MUR-region of CMV, in particular, of rodent, simian or human CMV, particularly preferred of human CMV, as S/MAR element, a vector nucleic acid according to the present invention, a cell or cell line according to the present invention for the expression of a molecule of interest, in particular, a nucleic acid molecule of interest or polypeptide of interest.

It has been recognized for the first time that the MUR-region of CMV represents a S/MAR region. S/MAR region in other microbes have been described rarely. That is, recently a S/MAR like DNA element was described from the gastrointestinal protozoan parasite *Giordia Lamblia,* BMC genomics, 2010, 11, 386. S/MAR are sequences found normally in eukaryotes, often associated with chromosomal regulation by bidirectional replication. Until now the human IFN-*β* gene derived S/MAR region has been used extensively in vector systems, but also other S/MAR elements have been investigated. (Zahn-Zabal and Kobr, 2001, J. Biot., 87, 29-42; Kim and Kim,2004, J. Biot. 107, 95-105; Girod and Mermod, 2003, New Comprehensive Biochemistry, 38, 359-379).

In a further aspect, the present invention provides a method for the production of a molecule of interest, in particular, a gene of interest or a polypeptide of interest. Said method comprises the step of transfecting a cell or cell line, preferably at mammalian cells or cell lines, with an expression vector containing the MUR-region of CMV. It is particularly preferred that the expression vector containing the MUR-region of CMV contains an MUR-region of rodent, simian or human CMV, particularly preferred of the human CMV as S/MAR region. Particularly preferred, the method is a method using a vector nucleic acid according to the present invention containing the MUR-region of CMV as S/MAR regions. The molecule of interest is produced by further steps known in the art.

Preferably, the method according to the present invention allows for the transient expression of a molecule of interest, wherein the expression vector is a vector nucleic acid of the following order: 5'-promoter, a molecule of interest or a gene encoding the molecule of interest, S/MAR consisting of the MUR-region of CMV, poly A wherein the promoter, the molecule of interest or the gene encoding the molecule of interest, S/MAR and poly A operably linked with each other.

In another preferred embodiment of the method according to the present invention, the method allows for the stable expression of a molecule of interest, wherein the expression vector is a vector nucleic acid as defined herein. The cell or cell line is transfected with the vector nucleic acid and the vector nucleic acid is either integrated to the host genome or is episomally replicating. Alternatively, a minicircle expression vector is formed in the cell or cell line allowing stable expression of the molecule of interest.

Finally, the present invention relates to a kit or system containing the vector nucleic acid, according to the present invention or a cell or cell line according to the present invention. The kit or system according to the present invention is particularly useful for the production of recombinant molecules of interest, in particular, nucleic acid molecules of interest or polypeptides of interest. The kit allows for transient or stable expression of said molecules of interest which is particularly desired in pharmaceutical and industrial industry.

In the following, the invention will be described by way of examples further without limiting the same thereto.

### Examples

### Example 1

Preparation of vector nucleic acids containing MUR derived from human CMV IE Region

The M and UR of hCMV was cloned upstream of the hCMV immediate early promoter in pCMVLUC, an expression plasmid carrying the hCMV immediate early promoter enhancer driving a firefly luciferase as reporter gene according to known methods. Luciferase expression was quantified 2 d after transfection (transient phase) using a standard luminometric assay and compared to the expression of the transfected construct lacking the MUR. Cotransfer of expression constructs using a neomycin resistance expression vector (pAG60, Colbere-Garapin, et al., 1981 J. Mol. Biol., 115, 1-14 and selection for stable integration using G418 resulted in clones 7-14 d post-transfection. Luciferase expression was determined from pooled clones arising after selection (stable expression upon integration). The 5' MUR element in standard expression plasmids increases gene expression when integrated into the chromosome especially in cell lines of murine and rat origin (like Ltk-, NIH3T and rat 1) but is not active in the transient phase of gene expression. This outcome represents a characteristic of S/MAR elements located upstream or flanking a transcription unit. A scheme of the vector nucleic acid is shown in figure 2a. In addition, data for the relative gene expression in transiently and stably transfected animal cell lines of expression vectors according to the present invention and vectors not containing MUR are shown in the table of figure 2a.

### Example 2

### Transient expression using minicircles

For transient expression the minicircles (MC) harboring a 3' MUR within the transcribed region (CMV), a cellular-S/MAR (M18, Broll et al. 2010, J. Mol. Biol. 395, 950-965) and a MUR-less MC (w/o), and all carrying by eukaryotic promoter followed by the eGFP reporter gene, were transfected into human embryo kidney HEK 293 cells and GFP expression (x Mean = average) was followed in the transient phase using flow cytometry. While the standard MCs loose gene expression as early as one day after transfection the viral S/MAR (MUR) extends the phase of transient expression at least up to day 4. Data are shown in figure 4.

### Example 3

### SIDD analysis

While efforts failed to identify really reliable S/MAR prediction upon sequence motifs, SIDD (stress induced duplex destabilisation) turned out to be a valuable in silico method to assess the presence of S/MARs in a given DNA region. SIDD is a statistical mechanical method to calculate the presence of mechanical stress-induced (twisting of DNA in supercoils) base-unpairing regions, which are a typical structural element describing S/MARs. In detail, the SIDD algorithm calculates the statistical mechanical equilibrium properties of the strand separation transition in a DNA sequence that is under negative super-helical stress (Benham et al., 1997, J. Mol Biol., 274; 181-196; Bi and Benham, 2004, Bioinformatics, 20, 1477-1479).SIDD was used to investigate the regions upstream of CMVIE enhancer promoters of different animal cytomegaloviruses. Peak values disclose the presence of S/MAR elements in the region 5' oft the immediate early promoter/ enhancers.

## Claims

1. A use of the MUR-region (modulator and unique region) of CMV (cytomegalomovirus) as a S/MAR element in expression vectors.

2. The use according to claim 1 in expression vectors for transient expression.

3. The use according to claim 1 in expression vectors for stable integration into the host chromosome or for stable expression upon extrachromosomal replication.

4. The use according to any one of the preceding claims wherein the S/MAR is the MUR-region of rodent, simian or human CMV, like any of of Seq. ID Nos. 1 to 5, in particular, of human CMV according to Seq. ID. No. 1.

5. A vector nucleic acid suitable for expressing at least one molecule of interest in a mammalian cell, comprising a S/MAR-region consisting of the MUR-region of CMV, in particular of rodent, simian or human CMV particularly preferred of human CMV, a promoter and the molecule of interest or a gene encoding the molecule of interest.

6. The vector nucleic acid according to claim 5 having the following order from the 5' end to the 3' end: 5'-S/MAR, promoter, molecule of interest or gene encoding the molecule of interest, poly A, S/MAR-3' whereby the S/MAR may be present either at the 5' end or 3' end or on both ends, preferably, wherein the S/MAR region consisting of the MUR region of CMV is present on both the 5' end and the 3' end.

7. The vector nucleic acid according to claim 5 having the following order from the 5' end to the 3' end: promoter, a molecule of interest or a gene encoding the molecule of interest, S/MAR consisting of the MUR-Region of CMV, poly A.

8. The vector nucleic acid according to any one of claims 5 to 7 wherein the molecule of interest is a gene encoding a polypeptide of interest, a gene encoding an antisense or silencer nucleic acid molecule, antibody, a DNA vaccine, a regulatory RNA molecule, or non-coding RNA molecule.

9. Cell or cell line containing a vector nucleic acid as defined in any one of claims 5 to 8 wherein said vector nucleic acid may be (i) stably integrated into the host genome or episomally replicating, or (ii) may be present as a minicircle expression vector for a stable or transient expression, preferably, wherein said cell or cell line is a mammalian cell line.

10. The use of the MUR-region of CMV as a S/MAR element according to any one of claims 1 to 4, a vector nucleic acid according to any one of claims 5 to 8 or a cell or cell line according to claim 9 for the expression of a molecule of interest, in particular, a nucleic acid molecule of interest or polypeptide of interest.

11. A method for the production of a molecule of interest, in particular, a gene of interest or a polypeptide of interest, comprising the step of transfecting a cell or cell line, preferably a mammalian cell or cell line, with an expression vector containing the MUR-region of CMV, in particular, of rodent, simian or human CMV, particularly preferred of human CMV, as a S/MAR-region, in particular with a vector nucleic acid according to any one of claims 5 to 8.

12. The method according to claim 11 for the transient expression of a molecule of interest, wherein the expression vector is a vector nucleic acid according to claim 7.

13. The method according to claim 11 for the stable expression of a molecule of interest, wherein the expression vector is a vector nucleic acid according to any one of claims 6 to 7.

14. A kit or system containing a vector nucleic acid according to claim 5 to 8 or a cell or cell line according to claim 9.

15. The kit or system according to claim 14 for use in the production of recombinant molecules of interest, in particular nucleic acid molecules of interest or polypeptides of interest.
